# EUROPEAN PATENT SPECIFICATION

(11) **EP 0 503 670 B1**
(45) Date of publication and mention of the grant of the patent: **22.05.1996**
(21) Application number: 92104412.9
(22) Date of filing: 13.03.1992
(51) Int. Cl.: A61M 5/172

(54) **Peristaltic intravenous infusion pump capable of displaying an application name selected**
Peristaltische intravenöse Infusionspumpe mit Anzeige einer gewählten Anwendung
Pompe d'infusion intraveineuse peristaltique capable d'afficher le nom de l'application sélectionnée

(30) Priority: 14.03.1991 JP 49898/91; 04.03.1992 JP 46776/92
(43) Date of publication of application: 16.09.1992
(73) Proprietor: SHARP KABUSHIKI KAISHA, Osaka 545 (JP); BAXTER INTERNATIONAL INC., Deerfield, IL 60015 (US)
(72) Inventor: Maeda, Akihiro, Souraku-gun, Kyoto-fu (JP); Lynn, Kenneth, McHenry, Illinois 60050 (US); Gluth, Michael, Arlington Heights, Illinois 60004 (US)
(74) Representative: TER MEER - MÜLLER - STEINMEISTER & PARTNER

(56) References cited:
- EP-A- 0 192 786
- EP-A- 0 319 268
- EP-A- 0 384 155
- WO-A-84/00894
- FR-A- 2 603 488
- US-A- 4 624 661

## Description

### BACKGROUND OF THE INVENTION

### 1. Field of the Invention

The present invention relates to a peristaltic intravenous infusion pump for medical use.

### 2. Description of the Prior Art

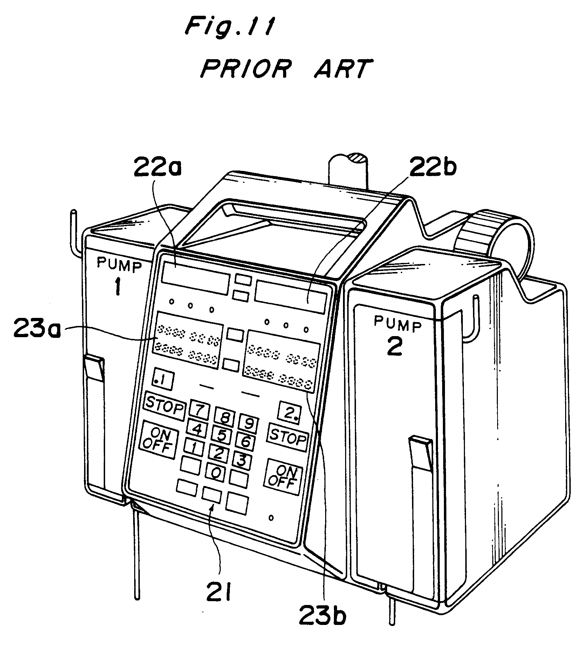

Fluid infusion pumps conventionally available are in general provided with a keyboard 21 and display units 22a, 22b, 23a, 23b, as shown in Fig. 11, and so adapted to allow the settings of the maximum rate of fluid infusion, maximum volume to be infused, occlusion sensing level, air bubble detection size, and the like to be set according to the purposes of applications (e.g. for infants or adults) and the places where they are used (e.g. operating room, surgical intensive care units).

The fluid infusion pumps are electromechanical, positive-pressure peristaltic type intravenous infusion devices having a pump mechanism, for use with a specified administration set. Programming for the devices is carried out through the keyboard 21. To enhance the safety of patients, the pump is provided with various types of alarm functions such as air bubble detection, upstream occlusion detection, and downstream occlusion detection.

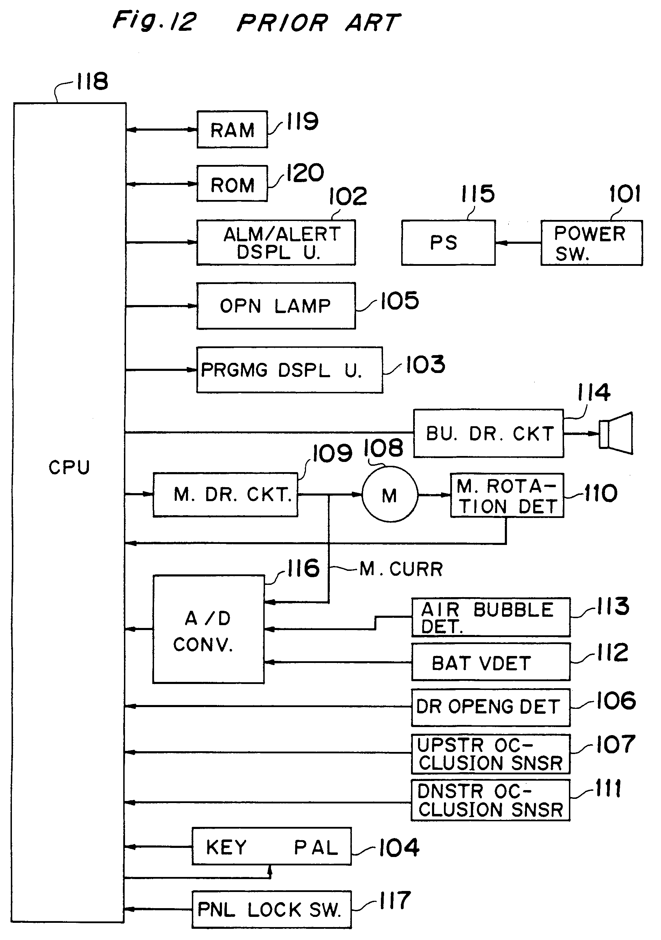

Fig. 12 is a block diagram of this type of a fluid infusion pump.

Referring to Fig. 12, a power switch, designated by numeral 101, turns ON the power when pressed. This operation is accompanied by a self test, which is automatically carried out temporarily. When the power switch 101 is pressed once more, the power turns OFF. An alarm/alert display unit, designated by 102, displays all the alarm and alert messages that the fluid infusion pump has detected. A programming display unit, designated by 103, displays all the program data concerning the fluid infusion such as rate, volume to be infused, and total volume infused, inputted for or collected by the fluid infusion pump. A key panel, designated by 104, has thereon numeric keys for inputting the rate and the volume to be infused, control keys for serving as an aid of input, a start key for starting the fluid infusion, a stop key for stopping the fluid infusion, and a call-up key for displaying the total volume infused and other data. An operation lamp, designated by 105, is a lamp that shows under which state it is currently among the alarm, fluid infusing operation, or alert state. A door opening detector, designated by 106, is a circuit for detecting whether or not the door has come into the open position, and necessary also for stopping the fluid infusion pump mechanism when the door is opened while the pump is driven and then generating the alarm. An upstream occlusion sensor, designated by 107, is used to detect the pressure-reduction state due to occurrence of any abnormality (e.g. clogged filter) in the administration set located between the administration bag and the fluid infusion pump and then generate an alarm so that the fluid infusion pump mechanism is stopped. A stepping motor, designated by 108, drives a linear peristaltic type fluid infusion pump mechanism specially designed for a specified administration set. This fluid infusion pump mechanism serves to press and release the tube for fluid infusion. A motor driving circuit, designated by 109, drives the stepping motor 108 in response to pulses outputted by a CPU 118. A motor-rotation detector, designated by 110, detects through the amount of motor rotation that a unit flow of fluid has taken place as a result of fluid conveyance by the fluid infusion pump mechanism, and then inform the CPU 118 of it. A downstream occlusion sensor, designated by 111, detects a pressure-rise state within a tube due to occurrence of any abnormality (e.g. occlusion) in the administration set located between the fluid infusion pump and the patient and generates an alarm to stop the fluid infusion pump mechanism. A battery-voltage detector, designated by 112, detects any battery-voltage drop of a lead battery (not shown) used as a backup of the AC power supply and generates an alarm to stop the fluid infusion pump mechanism. An air bubble detector, designated by 113, detects that air bubbles more than a prescribed amount has entered an administration tube, and generates an alarm while stopping the fluid infusion pump mechanism to thereby prevent the air bubbles from entering into the body. A buzzer driving circuit, designated by 114, generates a buzzer sound for informing doctors and nurses of the fact that the fluid infusion pump has come into the alarm/alert state. A power supply circuit, designated by 115, feeds power to all the circuits of the fluid infusion pump. An A/D converter, designated by 116, converts the voltage resulting from voltage conversion of the current applied to the motor 108, the air bubble detector output level, the battery voltage level, and the like into digital values, inputting them into the CPU 118. A panel lock switch, designated by 117, serves to render the panel key and the power key input-inhibited so that the fluid infusion pump will not be operated without permission by any person other than doctors or nurses. A CPU (Central Processing Unit), designated by 118, controls the operation of the fluid infusion pump. A RAM (memory section), designated by 119, includes all storage for various types of data used for arithmetic operation by the CPU 118. A ROM (program section), designated by 120, contains the program for operating the CPU 118.

Normally, in the conventional peristaltic intravenous infusion pump as described above, the maximum fluid infusion rate, the maximum volume to be infused, and the like are normally set by a biomedical engineer or serviceman in the hospital. However, in order to make it known what purpose and what place the pump unit has been set for by the aforementioned settings, it is necessary to attach a label onto the unit body saying the purpose and place. Because of this, if the label fails to be attached or is peeled off, there will arise a need in actual use of the unit for doctors and nurses to verify if the settings accord with the purpose of usage by the display unit or to relabel the unit each time the settings are changed, to a laborious disadvantage.

Also, the conventional peristaltic intravenous infusion pump is not subjected to a decision whether or not the settings such as the maximum fluid infusion rate and the maximum volume to be infused set by a biomedical engineer or serviceman in the hospital are appropriate for a selected application. This requires a further verification as to whether the settings are correctly made for a selected application. Moreover, if a label saying an incorrect purpose or place of usage different from those actually set should be attached on the fluid infusion pump unit because of misconfirmation or any other reason, it may matter critically.

In the conventional fluid infusion pump, the key panel is used to change and input the settings, such as the maximum fluid infusion rate and the maximum volume to be infused, for an application name selected according to the purpose and place of usage, or to change and input the settings, such as the maximum fluid infusion rate and the maximum volume to be infused, for the fluid infusion pump unit, or yet to select an application name in accordance with the purpose and place of usage out of a plurality of application names. Most hospitals each have tens or hundreds of fluid infusion pumps. It is very troublesome, tedious work to change and input the settings through the key panel with every one fluid infusion pump unit. There is also a possibility that some missetting may occur in changing and inputting the settings unit by unit. Furthermore, since the settings such as the maximum fluid infusion rate and the maximum volume to be infused can be changed and inputted through the key panel, there is a danger that these settings may be changed by some person who is not authorized to do that.

EP-A-0319268 discloses a medication infusion system including an interface which controls physically separate and discrete pumps having respective parameter settings. These parameter settings/values are set at the factory as default settings. An operator is simply limited to selecting a particular clinical device type, and while the clinician can visually check the device settings, he cannot change them. Changes can only be made with the use of specialized equipment by biomedical engineers or by the manufacturer. The pump itself does not include a display for displaying the currently programmed pump parameters and an input device for permitting an operator to select an application name and selectively modify infusion-related parameter values of the pump. Also, no means is disclosed for automatically assuring that the selectively modified pump parameter values are within a predetermined range.

EP-A-0192786 discloses a processor-controlled angiographic injector device for injecting into a patient contrast media at a controlled rate and pressure during x-ray photography. This known device comprises a syringe containing liquid contrast media, drive means for forcing the media from the syringe, and a processor control system for calculating injection control signals and for controlling the delivery of the contrast media. In addition, a preprogrammed storage module is provided for storing routine injection parameters.

### SUMMARY OF THE INVENTION

The present invention has been developed with a view to substantially solving the above described disadvantages and has for its essential object to provide a peristaltic intravenous infusion pump which allows simple verification as to what purpose and what place the settings have been made for, without labeling the unit or verifying for actual use the settings such as the maximum fluid infusion rate and the maximum volume to be infused.

Another object of the present invention is to provide a peristaltic intravenous infusion pump which eliminates the need of checking if the settings are made correctly for the selected application.

A still further object of the present invention is to provide a peristaltic intravenous infusion pump capable of preventing a danger that the settings may be changed by any unauthorized person, with an arrangement that change and input of the settings as well as selection of application names can be effected from an external control unit such as a computer, thereby facilitating the operation and preventing occurrence of any missetting.

In order to achieve the above objectives, a peristaltic intravenous infusion pump is provided having the features of appended claim 1.

In the peristaltic intravenous infusion pump of a first embodiment, the maximum fluid infusion rate, the maximum volume to be infused, and the like can be set according to application such as the purpose and place of usage, while an application name corresponding to the contents of the settings is selected by the selection means from among a plurality of application names stored in the storage means, and the selected application name is displayed onto the display means. Therefore, the application of the peristaltic intravenous infusion pump can be simply verified by viewing the application name displayed on the display means, saving the trouble of attaching a label saying the application name onto the pump or verifying the settings when the pump is put into use. Moreover, even when the contents of the settings are changed, all that is additionally required is to change the display of the application name in correspondence to the contents of the setting, saving the trouble of replacing the label or other tasks, which have conventionally been required.

In addition, the decision means serves to decide whether or not a setting set by the setting means is appropriate for the application name selected by the selection means by comparing it to the corresponding setting stored in the setting storage means, thus facilitating correct setting for the selected application. Moreover, as a result of the decision, if the setting set by the setting means is inappropriate for the selected application name, it alarms the setter of it, thereby enabling him or her to notice a missetting or an inappropriate selection of application name before it matters. Therefore, there is no possibility that an incorrect label saying any purpose or place of usage different from those actually set according to the purpose and place of usage may be attached onto the body of the peristaltic intravenous infusion pump.

In the peristaltic intravenous infusion pump of a second embodiment of the invention, the data processing means serves to transmit settings set by the setting means, settings and applications stored in the setting storage means to the external control unit via the communication means, or to change the settings set by the setting means or settings and applications stored in the setting storage means, in response to instructions from the external control unit via the communication means. Accordingly, since the settings and applications can be set or changed by the external control unit, there is no need of implementing such setting and changing with individual peristaltic intravenous infusion pumps unit by unit, saving time and labor of operation. Also, if a plurality of peristaltic intravenous infusion pumps are connected with one external control unit, it is possible to set and change the settings for each peristaltic intravenous infusion pump at the same time. If the external control unit is adapted to record the settings directed to one of the peristaltic intravenous infusion pumps, it is possible to set the same settings with every peristaltic intravenous infusion pump even though the settings are set or changed for individual units of the peristaltic intravenous infusion pumps. Furthermore, if the setting and change of the settings are associated with the body of the peristaltic intravenous infusion pump, there will be no possibility that they may be changed by any unauthorized person. It further becomes feasible to remotely control the peristaltic intravenous infusion pump, for example by installing the pump in a hospital room and the external control unit in the nurse station.

### BRIEF DESCRIPTION OF THE DRAWINGS

The present invention will become more fully understood from the detailed description given hereinbelow and the accompanying drawings which are given by way of illustration only, and thus are not limitative of the present invention, and wherein:
Fig. 1 is a block diagram of an essential part of a peristaltic intravenous infusion pump according to a first embodiment of the present invention;
Fig. 2 is a block diagram of an essential part of a peristaltic intravenous infusion pump according to a second embodiment of the present invention;
Fig. 3 is a view showing an example of storage contents of an application-related set data storage area of the peristaltic intravenous infusion pump of the second embodiment of the invention;
Fig. 4 is a view showing an example of storage contents of a pump-related set data storage area of the second peristaltic intravenous infusion pump of the second embodiment;
Fig. 5 is a flowchart showing an example of the rewrite of the application-related set data storage area of the peristaltic intravenous infusion pump of the second embodiment;
Fig. 6 is a flowchart showing an example of the selection of an application name and the rewrite of the pump-related set data storage area of the peristaltic intravenous infusion pump of the second embodiment;
Fig. 7 is a view showing an example of error message display in the second embodiment;
Fig. 8 is a block diagram of an essential part of a peristaltic intravenous infusion pump according to the third embodiment of the present invention;
Figs. 9 and 10 are flowcharts showing the operation of the peristaltic intravenous infusion pump of the third embodiment of the present invention;
Fig. 11 is an outside view of the conventional peristaltic intravenous infusion pump; and
Fig. 12 is a block diagram showing a construction that peristaltic intravenous infusion pumps generally have.

### DETAILED DESCRIPTION OF THE PREFERRED EMBODIMENTS

The following description is made on the assumption that peristaltic intravenous infusion pumps of the following embodiments of the present invention have components as shown in Fig. 12 as a basic construction as well as a panel similar to that of Fig. 11.

### First Embodiment

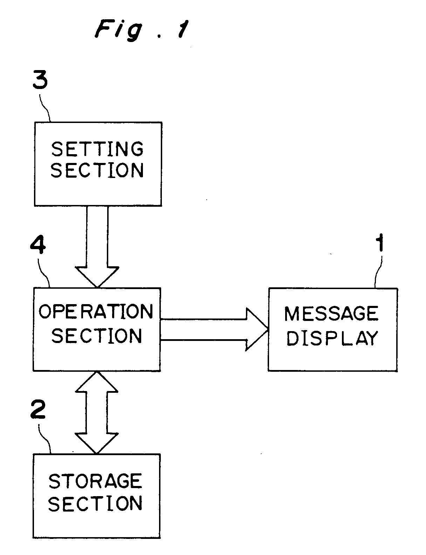

The peristaltic intravenous infusion pump of this embodiment is so adapted that the settings of a maximum fluid infusion rate, a maximum volume to be infused, and others can be changed according to application such as the purpose and place of usage. This peristaltic intravenous infusion pump, as shown in Fig. 1, has a message display 1 as display means capable of displaying various types of messages, a storage section 2 as storage means having a rewritable memory in which a plurality of application names are stored, a setting section 3 as both selection means for selecting one from among the plurality of application names stored in the storage section 2 and setting means for instructing any rewrite of the contents of the storage section 2, and an operation section 4 for accessing the storage section 2 for an application name selected by the setting section 3 and allowing the application name to be displayed on the message display 1 or for rewriting the storage contents of the storage section 2 in response to an instruction of the setting section 3.

Examples of the application names to be stored in the storage section 2 are listed below:
(1) NICU (ICU for the new-born)
(2) PICU (ICU for infants)
(3) MED·SURGICAL (surgery)
(4) TRAUMA·BURN UNIT (traumas/burns)
(5) OPER ROOM (operating room)
(6) CARDIAC·ICU (circulatory organ ICU)
(7) SURGICAL·ICU (surgery ICU)
(8) OTHER ICU (other ICUs)
(9) ONCOLOGY (tumorous surgery)

The display of these application names onto the message display 1 is effected by selecting an application name corresponding to the setting contents with the setting section 3 when a biomedical engineer or serviceman of the hospital sets the maximum fluid infusion rate and others. The application name selected will be displayed on the message display 1, for example when the power for the pump is turned ON or while the power is OFF or when a specific key is operated, as required. Accordingly, it is possible for the doctor or nurse who actually uses the peristaltic intravenous infusion pump to know what purpose the pump has been set for by viewing the application name displayed on the message display 1, thereby facilitating correct setting for the selected application when he or she uses the pump. Also, it is possible to prevent misuse of the pump, such as putting the pump set for adults into use for infants. Moreover, the storage contents of the storage section 2 can be rewritten with the setting section 3, allowing the pump to be applied to a variety of applications in a simple manner.

### Second Embodiment

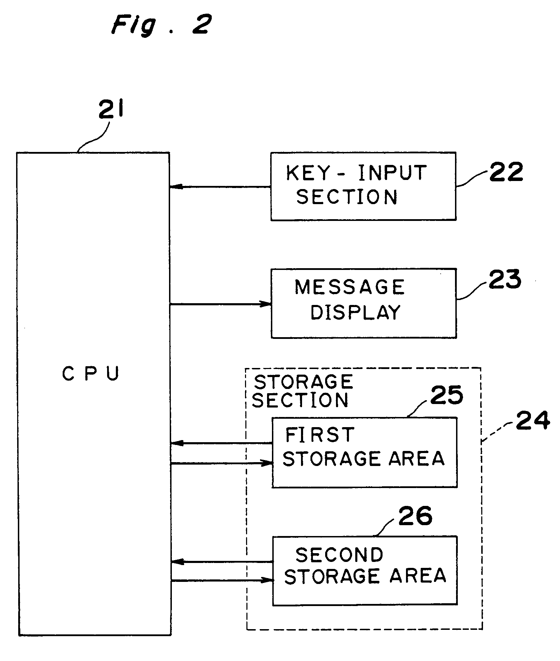

The peristaltic intravenous infusion pump of this embodiment, as shown in Fig. 2, has a message display 23 as a display means capable of displaying various types of messages, and a storage section 24, as a setting storage means having a rewritable memory. The storage section 24 has a first storage area 25 (referred to as a pump-related set data storage area below) which stores therein set data that has been set with respect to the pump, and a second storage area 26 (referred to as an application-related set data storage area below) which stores therein a plurality of application names and set data for each application name. The peristaltic intravenous infusion pump also has a key-input section 22 as both a selection means for selecting one from among the plurality of application names stored in the storage section 24 and a setting means for instructing the rewrite of the contents of the storage section 24, and a CPU 21 as a decision means for accessing the storage section 24 for the application name selected by the key-input section 22 and displaying it onto the message display 23, or rewriting the contents of the storage section 24 in response to an instruction from the key-input section 22, or comparing the settings of the pump-related set data storage area 25 with those of the application-related set data storage area 26.

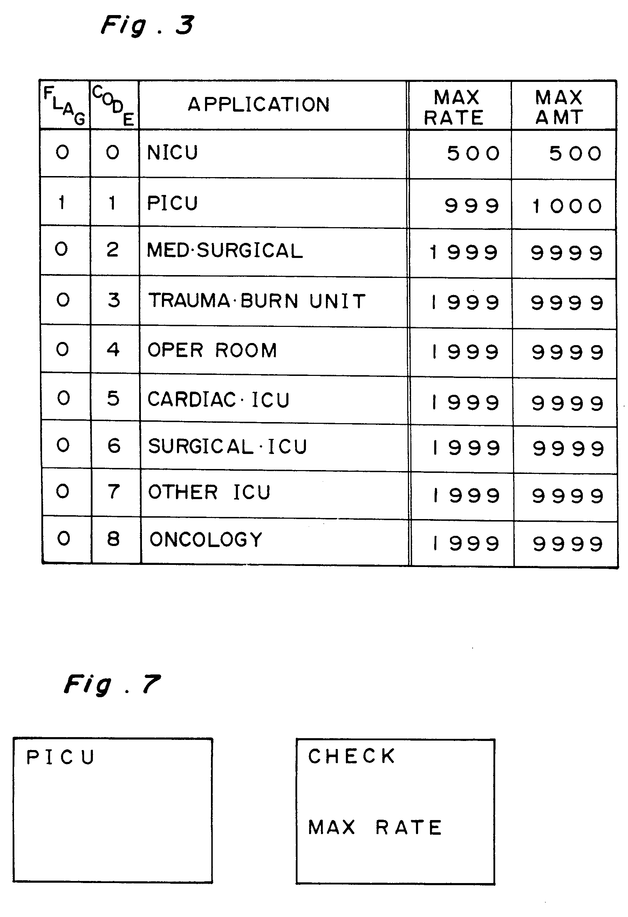

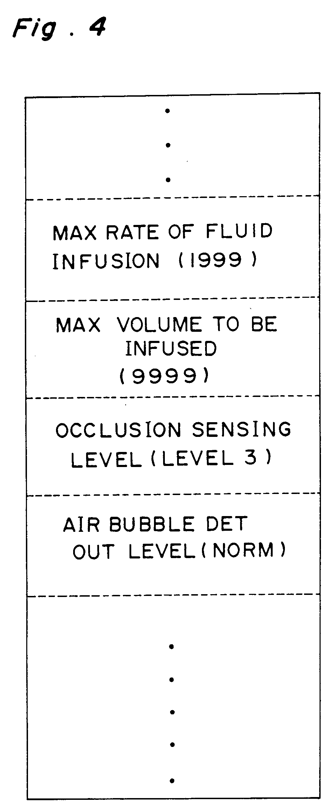

An example of set data of the pump-related set data storage area 25 and that of the application-related set data storage area 26 are shown in Fig. 4 and Fig. 3, respectively. Although Fig. 3 has only the settings of the maximum fluid infusion rate and the maximum volume to be infused with respect to each application, it is also possible to further set the occlusion sensing level, air bubble detection size, and the like.

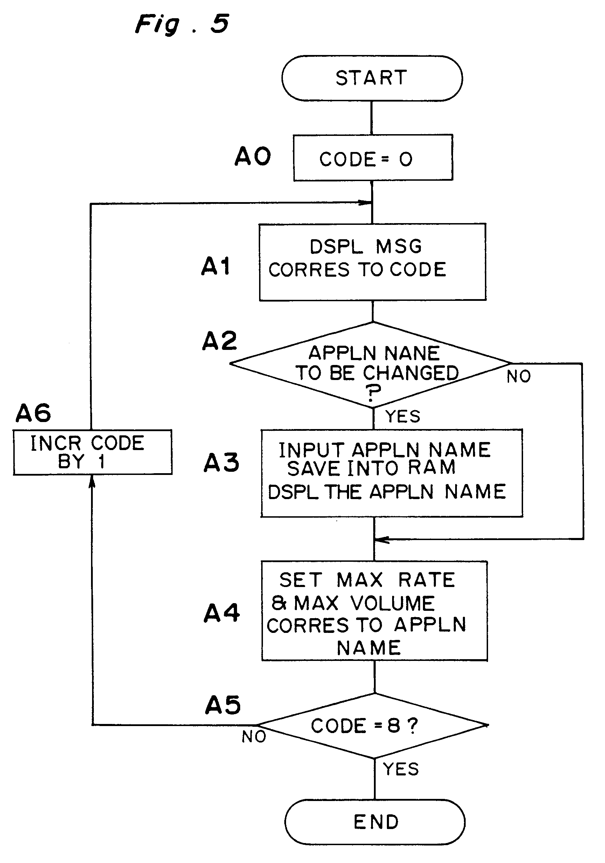

Next, following the flowchart in Fig. 5, rewriting of set data in the application-related set data storage area 26 is described below.

First, with the pump unit in the power-OFF state, an ON/OFF key is pressed while a specific key (e.g. PUMP1 key) is pressed, thereby entering the application-related set data rewrite mode. The code at this time is 0. With step A0 succeeded by step A1, message data corresponding to the code 0 is displayed onto the message display 23. Then it is decided at step A2 whether the application name is changed or not. To change it, the sequence goes to step A3; otherwise, it goes to step A4. At step A3, an application name is inputted through the key-input section 22; message data (application name) corresponding to the code in Fig. 3 is rewritten and saved; and the rewritten application name is displayed onto the display. At step A4, the maximum fluid infusion rate and the maximum volume to be infused corresponding to the application displayed on the display 23 are inputted in sequence through the key-input section 22, and the set data in Fig. 3 is rewritten and saved. Subsequently at step A5, it is decided whether or not the processing from step A1 to step A4 is completed for all the codes; if not, then at step A6, 1 is added to the code, the processing from step A1 to step A4 is repeated with respect to the resulting code.

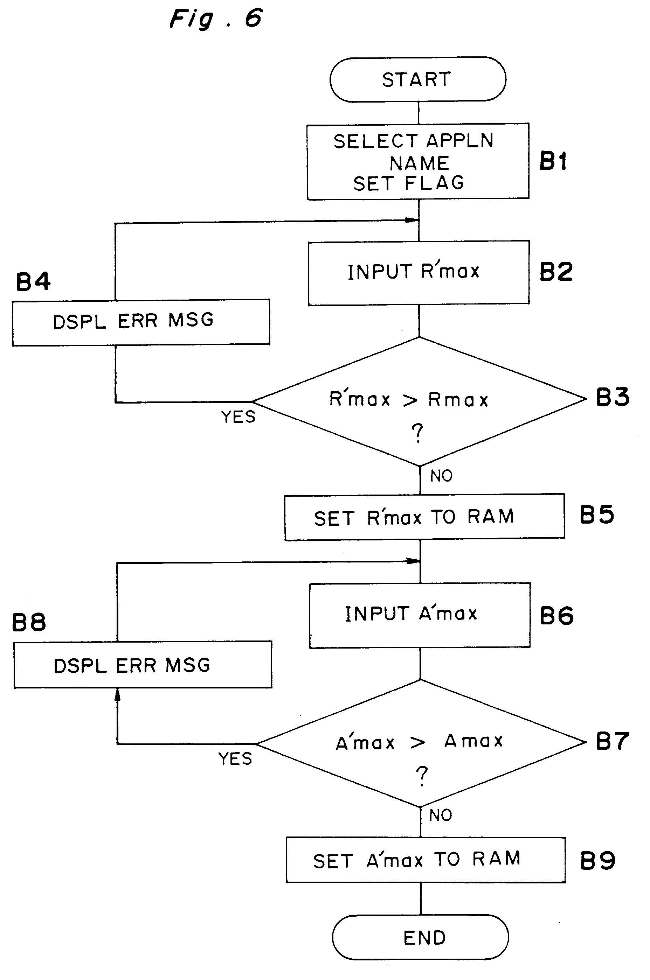

Following to the flowchart in Fig. 6, now the selection of an application name and the rewrite of the pump-related set data storage area 25 are described.

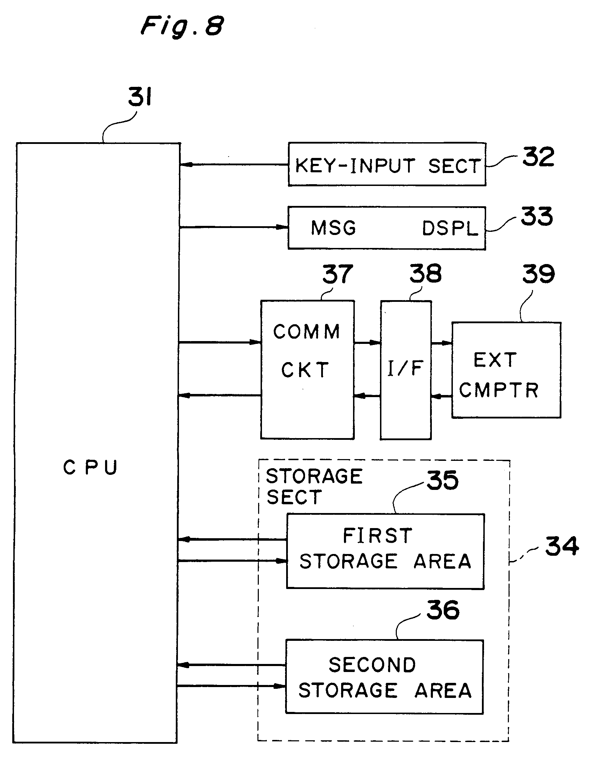

First, with the peristaltic intravenous infusion pump unit in the power-OFF state, the ON/OFF key is pressed while a specific key (e.g. PANEL-LOCK or STOP key) is pressed, thereby entering the application-name selection and pump-related set data rewrite mode. First at step B1, message data (application name) is selected by selecting a code shown in Fig. 3 through the key-input section 22, a flag indicating the selection being set. Then at step B2, a maximum fluid infusion rate R'max to be set is inputted through the key-input section 22. Thereafter, at step B3, it is decided whether or not the value R'max inputted at step B2 is greater than a maximum fluid infusion rate Rmax for the application to which the flag is set (selected). As a result of the decision, if the value R'max inputted at step B2 is greater than the maximum fluid infusion rate Rmax for the selected application, the sequence goes to step B4, where such an error message as shown in Fig. 7 is displayed onto the message display 23. On the other hand, if the input value R'max is equal to or smaller than that Rmax for the selected application as a result of the decision, the input value R'max is decided to be appropriate for the application. Then, at step B5, the input value is saved into the pump-related set data storage area 25 (RAM). Also with the maximum volume to be infused, the processing from step B6 to B9 is effected in the same manner as in the processing from B2 to B5 with the maximum fluid infusion rate. In Fig. 6, A'max is a maximum volume to be infused inputted through the key input 22 be set and Amax is a maximum volume to be infused for an application selected.

Although in this example only the settings of the maximum fluid infusion rate and the maximum volume to be infused are checked at the time of input, other set data can also be checked likewise.

As described above, according to this embodiment, if the maximum fluid infusion rate or the maximum volume to be infused inputted from the key-input section 22 is inappropriate for the selected application, a relevant error message is displayed. Hence any missetting or inappropriate selection of application can be prevented beforehand.

As for the display of any selected application name, it is possible to display it onto the message display 23 as required, for example when the power for the pump is turned ON, or while the power is OFF, or when a specific key is operated. Accordingly, if a desired application name is being displayed, it is unnecessary to verify the settings when the pump is put into use, as in the first embodiment of the present invention, and moreover any misuse of the pump can be prevented beforehand.

### Third Embodiment

The peristaltic intravenous infusion pump of this embodiment, as shown in Fig. 8, has a message display 33 capable of displaying various types of messages, and a storage section 34 as a setting storage means having a rewritable memory. The storage section 34 has a first storage area (referred to as a pump-related set data storage area below) 35, which stores therein set data that has been set with respect to the pump, and a second storage area (referred to as an application-related set data storage area below) 36, which stores therein a plurality of application names and set data for each application name. The peristaltic intravenous infusion pump also has a communication circuit 37 and an interface (I/F) 38 as a communication means necessary for communication between the pump and an external computer 39, a key-input section 32 as a setting means which can put the peristaltic intravenous infusion pump into a communication mode with the external computer 39, and a CPU 31 as data processing means for rewriting the contents of the pump-related and application-related set data storage areas 35 and 36 in response to an instruction from the external computer 39, or comparing the data received from the external computer 39 with the settings of the storage areas 35 and 36, or transmitting data within the storage section 34 to the external computer 39.

The storage section 34 being identical with the storage section 24 of the second embodiment, set data as exemplified in Fig. 4 and Fig. 3 are stored in the storage areas 35 and 36.

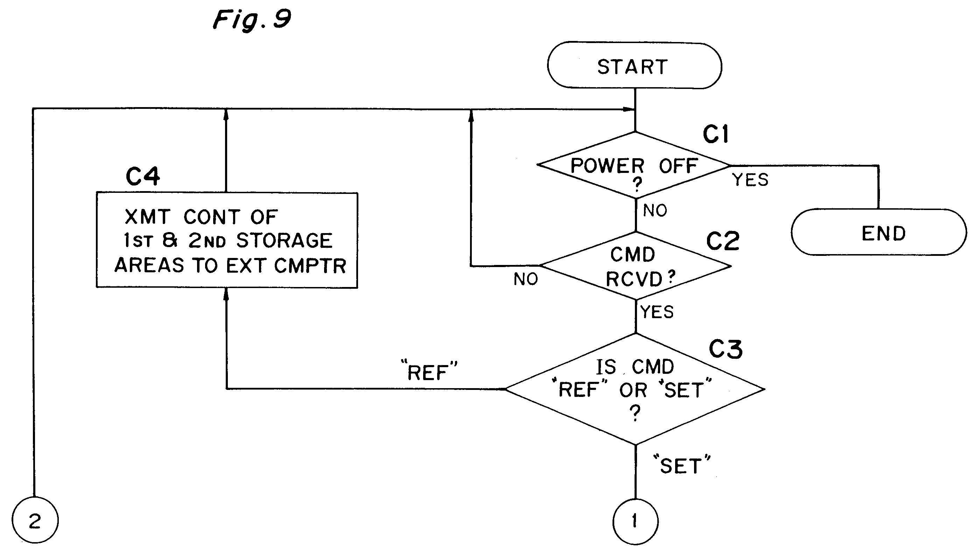

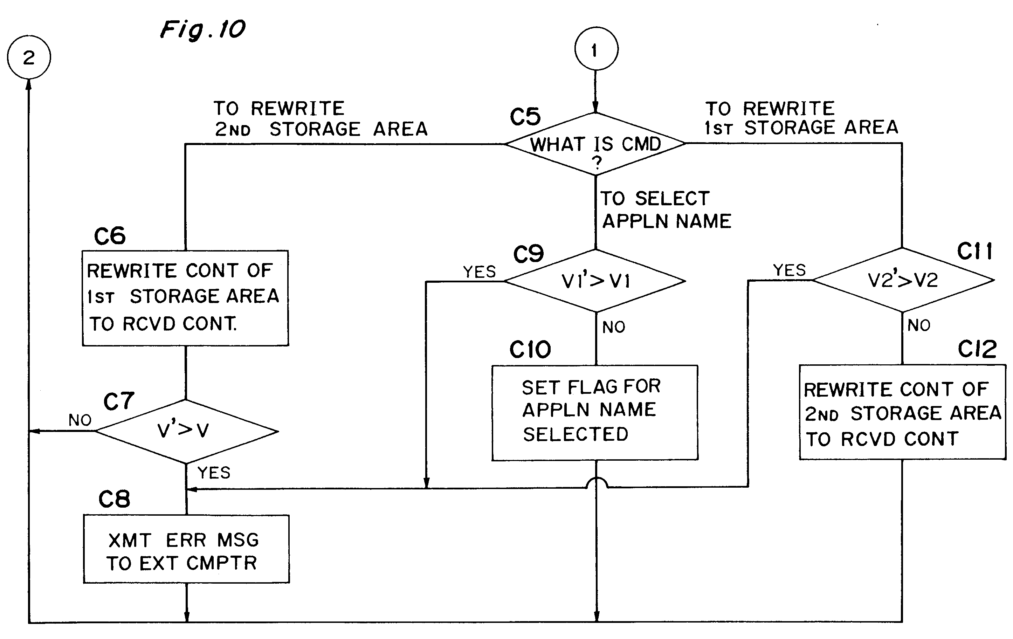

Now following the flow of operation shown in Fig. 9 and Fig. 10, the present embodiment is detailed below.

First, the pump is brought into the communication mode through the key-input section 32. The CPU 31 checks the power supply for the pump at step C1. If the power is OFF, the processing is terminated; otherwise, at the next step C2, it is checked whether or not any command has been received from the external computer 39. If no command has yet been received, step C1 and step C2 are repeatedly executed. If some command has been received, the CPU 31 goes to step C3, where it is decided whether the received command is a "reference" command for causing the CPU 31 to refer to the contents of the storage section 34 or a "set" command for causing the CPU 31 to rewrite the contents of the storage section 34.

If the received command is the "reference" command, the CPU 31 goes to step C4, where it reads set data from the pump-related set data storage area 35 and the application-related set data storage area 36 within the storage section 34, and writes the read contents into the communication circuit 37, thereby transmitting it to the external computer 39.

On the other hand, if the received command is the "set" command, the CPU 31 goes to step C5. At step C5, it is decided whether the "set" command is one that leads to the rewrite of the second storage area 36 or another that leads to the selection of an application name, or yet another that leads to the rewrite of the first storage area 35. In compliance with the decision result, the CPU 31 goes to step C6, C9, or C11 for each case.

At step C6, set data, as shown in Fig. 3, that is stored in the application-related set data storage area 36 is rewritten into data received from the external computer 39. Then at step C7, a comparison is made between the settings (represented by V here) such as the maximum fluid infusion rate and the maximum volume to be infused for an application being currently selected by the pump and the current settings (represented by V' here) such as the maximum fluid infusion rate and the maximum volume to be infused for the pump. As a result of the comparison, if there is any inconsistency (in the case of the maximum fluid infusion rate and the maximum volume to be infused, if the current setting V' of the maximum fluid infusion rate or the maximum volume to be infused for the pump has become greater than the setting V of the maximum fluid infusion rate or the maximum volume to be infused for the application being currently selected by the pump), the CPU 31 goes to step C8, where the contents of the inconsistency or the like are transmitted as an error message to the external computer 39, returning to step C1. If there is no inconsistency, the CPU 31 returns to step C1.

At step C9, a comparison is made between the settings (represented by V1 here) such as the maximum fluid infusion rate and the maximum volume to be infused for an application that is about to be selected by the external computer 39 and the current settings (represented by V1' here) such as the maximum fluid infusion rate and the maximum volume to be infused for the pump. As a result of the comparison, if there is any inconsistency (in the case of the maximum fluid infusion rate or the maximum volume to be infused, if the current setting V1' of the maximum fluid infusion rate or the maximum volume to be infused for the pump has become greater than the setting V1 of the maximum fluid infusion rate or the maximum volume to be infused for the application that is about to be selected by the external computer 39), the CPU 31 goes to step C8, where the contents of the inconsistency or the like are transmitted as an error message to the external computer 39, returning to step C1. If there is no inconsistency, the CPU 31 goes to step C10, where the application name selected by the external computer 39 is selected and set (a flag (shown in Fig. 3) corresponding to the selected application is set), returning to step C1.

At step C11, a comparison is made between the settings (represented by V'2 here) of the maximum fluid infusion rate and the maximum volume to be infused that the external computer 39 is about to set to the pump-related set data storage area 35 and the settings (represented by V2 here) of the maximum fluid infusion rate and the maximum volume to be infused for the application currently selected. As a result of the comparison, if there is any inconsistency (in the case of the maximum fluid infusion rate or the maximum volume to be infused, if the value V'2 of the maximum fluid infusion rate or the maximum volume to be infused that the external computer 39 is about to set to the pump-related set data storage area 35 is greater than setting V2 of the maximum fluid infusion rate or the maximum volume to be infused for the application currently selected by the pump), the CPU 31 goes to step C8, where the contents of the inconsistency or the like are transmitted as an error message to the external computer 39, returning to step C1. If there is no inconsistency, the CPU 31 goes to step C12, where set data as shown in Fig. 4 stored in the pump-related set data storage area 35 is rewritten into the data received from the external computer 39, returning to step C1.

As described above, since the external computer 39 allows the settings such as the maximum fluid infusion rate and the maximum volume to be infused to be changed or inputted therethrough, it is unnecessary to treat the peristaltic intravenous infusion pump to execute change or input of the settings. Also, connecting a plurality of peristaltic intravenous infusion pumps with the external computer 39 allows the settings for every pump to be changed or inputted at the same time; otherwise, even if the settings are changed or inputted for each one unit of the peristaltic intravenous infusion pumps, the same settings can be ensured with simplicity. Further, it is also made feasible to remotely control the pumps installed in respective hospital rooms by means of the external computer 39 installed in the nurse station. Moreover, inhibiting change and input of the settings from the key-input section 32 will serve to prevent the settings from being changed by any unauthorized person.

The invention being thus described, it will be obvious that the same may be varied in many ways. For example, while the specific embodiment of the present invention has been described for use in an electromechanical intravenous infusion apparatus of the positive-pressure peristaltic type, use of the present invention is not intended to be limited to this type of pumping apparatus. Additionally, examples of particular flow rates, etc., contained in this application are intended for illustrative purposes only. The present device is not intended to be limited to use in such ranges. Such variations are not to be regarded as a departure from the invention as claimed, and all such modifications as would be obvious to one skilled in the art are intended to be included within the scope of the following claims.

## Claims

1. A peristaltic intravenous infusion pump, comprising:
a display (1, 23, 33);
a memory (2, 24, 34) for storing a plurality of application names corresponding to different medical operations and associated preset infusion-related parameter values of the pump such as a maximum fluid infusion rate and maximum volume to be infused;
means for displaying on the display a currently selected application name and its corresponding preset parameter values in response to initial activation of the pump such that an operator may immediately visually confirm the purpose, place and preset parameter values of the pump;
an input device (22, 32) for permitting an operator to select any one of the plurality of application names and to selectively modify its associated preset infusion-related parameter values;
decision means (21) for automatically assuring that the selectively modified parameter values are within a predetermined range; and
control means (118) for operating the infusion pump in accordance with the infusion-related parameter values associated with the currently selected application.

2. The peristaltic intravenous infusion pump as claimed in claim 1, further comprising:
a setting storage means for storing the modified parameter values input by the input device for each of the different medical operations corresponding to the application names, and
the decision means including means for reading the present parameter values from the memory corresponding to a selected application, comparing the preset parameter values with the modified personal parameter values, and deciding whether or not the modified parameter values lie within the determined range for the selected application name.

3. The peristaltic intravenous infusion pump as claimed in claim 2, further comprising:
a communication means for exchanging data including infusion-related pump parameter values and corresponding application names with an external control unit, and
a data processing means, in response to an instruction received from the external control unit via the communication means, transmitting the preset parameter values and the application names stored in the memory to the external control unit via the communication means and modifying the proset parameter values and application names based on data received from the external control unit.

4. The peristaltic intravenous infusion pump as claimed in claim 1, wherein the initial activation of the pump includes application of power to the pump.

5. The peristaltic intravenous infusion pump as claimed in claim 1, wherein the initial activation of the pump includes actuation of a switch or key on the input device by an operator.

6. The peristaltic intravenous infusion pump as claimed in claim 1, wherein each application name stored in the memory is assigned a corresponding code and a specific maximum fluid infusion rate and a maximum infusion volume associated with that application name.

7. The peristaltic intravenous infusion pump as claimed in claim 6, wherein an operator may select an application name by selecting its corresponding code via the input device.

8. The peristaltic intravenous infusion pump as claimed in claim 2, further comprising:
means for generating an error message when the decision means decides that the modified parameter values are outside the determined range for the selected application name.

## Patentansprüche

1. Peristaltische intravenöse Infusionspumpe mit
- einer Anzeige (1, 23, 33);
- einem Speicher (2, 24, 34) zum Einspeichern mehrerer Anwendungsnamen, die verschiedenen medizinischen Vorgängen entsprechen, und zugehöriger, voreingestellter infusionsbezogener Parameterwerte der Pumpe wie der maximalen Flüssigkeitsinfusionsrate und dem maximalen Infusionsvolumen;
- einer Einrichtung zum Anzeigen des Namens der aktuell ausgewählten Anwendung auf der Anzeige sowie der ihr zugehörigen voreingestellten Paramterwerte, auf die anfängliche Aktivierung der Pumpe hin, so daß die Bedienperson unmittelbar visuell den Zweck, den Ort und die voreingestellten Parameterwerte der Pumpe klarstellen kann;
- einer Eingabevorrichtung (22, 32), mit der die Bedienperson jeden der mehreren Anwendungsnamen auswählen kann und die zugehörigen, voreingestellten, infusionsbezogenen Parameterwerte wahlweise modifizieren kann;
- einer Entscheidungseinrichtung (21) zum automatischen Sicherstellen, daß die wahlweise modifizierten Parameterwerte innerhalb eines vorgegebenen Bereichs liegen; und
- einer Steuereinrichtung (118) zum Betreiben der Infusionspumpe entsprechend den infusionsbezogenen Parameterwerten, wie sie der aktuell ausgewählten Anwendung zugeordnet sind.

2. Peristaltische intravenöse Infusionspumpe nach Anspruch 1, ferner mit:
- einer Einstellspeichereinrichtung zum Einspeichern der modifizierten, durch die Eingabevorrichtung eingegebenen Parameterwerte für jeden der verschiedenen medizinischen Vorgänge, entsprechend den Anwendungsnamen; und
- wobei die Entscheidungseinrichtung eine Einrichtung für folgendes aufweist: Auslesen der voreingestellten Parameterwerte aus dem Speicher, entsprechend der ausgewählten Anwendung, Vergleichen der voreingestellten Parameterwerte mit den modifizierten, persönlichen Parameterwerten und Entscheiden, ob die modifizierten Parameterwerte innerhalb des vorgegebenen Bereichs für den ausgewählten Anwendungsnamen liegen oder nicht.

3. Peristaltische intravenöse Infusionspumpe nach Anspruch 2, ferner mit:
- einer Kommunikationseinrichtung zum Austauschen von Daten einschließlich infusionsbezogener Pumpenparameterwerte und entsprechender Anwendungsnamen mit einer externen Steuereinheit; und
- einer Datenverarbeitungseinrichtung, die auf eine von der externen Steuereinheit über die Kommunikationseinrichtung empfangene Anweisung hin die voreingestellten Parameterwerte und die Anwendungsnamen, wie im Speicher abgespeichert, über die Kommunikationseinrichtung an die externe Steuereinheit überträgt und die voreingestellten Parameterwerte und die Anwendungsnamen auf Grundlage von von der externen Steuereinheit empfangenen Daten modifiziert.

4. Peristaltische intravenöse Infusionspumpe nach Anspruch 1, bei der die anfängliche Aktivierung der Pumpe das Anlegen von Spannung an die Pumpe umfaßt.

5. Peristaltische intravenöse Infusionspumpe nach Anspruch 1, bei der die anfängliche Aktivierung der Pumpe die Betätigung eines Schalters oder Schlüssels an der Eingabevorrichtung durch die Bedienperson umfaßt.

6. Peristaltische intravenöse Infusionspumpe nach Anspruch 1, bei der jedem im Speicher abgespeicherten Anwendungsnamen ein entsprechender Code zugeordnet ist und eine spezielle maximale Flüssigkeitsinfusionsrate und ein maximales Infusionsvolumen in Zuordnung zu diesem Anwendungsnamen vorhanden sind.

7. Peristaltische intravenöse Infusionspumpe nach Anspruch 6, bei der die Bedienperson einen Anwendungsnamen dadurch auswählen kann, daß sie den zugehörigen Code über die Eingabevorrichtung auswählt.

8. Peristaltische intravenöse Infusionspumpe nach Anspruch 2, ferner mit einer Einrichtung zum Erzeugen einer Fehlermeldung, wenn die Entscheidungseinrichtung entscheidet, daß die modifizierten Parameterwerte außerhalb des für den ausgewählten Anwendungsnamen bestimmten Bereichs liegen.

## Revendications

1. Pompe péristaltique d'injection intraveineuse comprenant :
un dispositif d'affichage (1,23,33);
une mémoire (2,24,34) pour mémoriser une pluralité de noms d'applications correspondant à différentes opérations médicales et à des valeurs associées préréglées de paramètres de la pompe, liés à l'injection, comme par exemple le débit maximum du fluide d'injection et le volume maximum devant être injecté.
des moyens pour afficher, sur le dispositif d'affichage, un nom d'application actuellement sélectionné et les valeurs correspondantes préréglées de ses paramètres, en réponse à une activation initiale de la pompe de sorte qu'un opérateur peut immédiatement confirmer visuellement le but, l'emplacement et les valeurs préréglées des paramètres de la pompe;
un dispositif d'entrée (22,32) permettant à l'opérateur de sélectionner n'importe lequel de la pluralité de noms d'applications et de modifier de façon sélective les valeurs préréglées de paramètres liés à l'injection, qui sont associés aux applications;
des moyens de décision (21) pour garantir automatiquement que les valeurs modifiées sélectivement des paramètres se situent dans une gamme prédéterminée; et
des moyens de commande (118) pour faire fonctionner la pompe d'injection en fonction des valeurs de paramètres liés à l'injection et associés à l'application actuellement sélectionnée.

2. Pompe péristaltique d'injection intraveineuse selon la revendication 1, comportant en outre :
des moyens de mémorisation de réglages servant à mémoriser les valeurs modifiées des paramètres, introduites par le dispositif d'entrée pour chacune des différentes opérations médicales correspondant aux noms d'applications; et
les moyens de décision contenant des moyens pour lire dans la mémoire les valeurs actuelles de paramètres correspondant à une application sélectionnée, comparer les valeurs préréglées des paramètres aux valeurs modifiées des paramètres personnels et décider si les valeurs modifiées des paramètres se situent ou non dans la gamme déterminée pour le nom d'application sélectionné.

3. Pompe péristaltique d'injection intraveineuse selon la revendication 2, comprenant en outre :
des moyens de communication pour échanger des données incluant des valeurs de paramètres de la pompe, liés à l'injection, et des noms d'applications correspondants avec une unité de commande externe, et
des moyens de traitement de données qui, en réponse à une instruction reçue de la part de l'unité de commande externe par l'intermédiaire des moyens de communication, transmettent les valeurs préréglées des paramètres et les noms d'application mémorisés dans la mémoire à l'unité de commande externe par l'intermédiaire des moyens de communication, et modifient les valeurs préréglées des paramètres et les noms d'applications sur la base de données reçues à partir de l'unité de commande externe.

4. Pompe péristaltique d'injection intraveineuse selon la revendication 1, dans laquelle l'activation initiale de la pompe inclut l'application d'une énergie à la pompe.

5. Pompe péristaltique d'injection intraveineuse selon la revendication 1, dans laquelle l'activation initiale de la pompe inclut l'actionnement, par un opérateur, d'un interrupteur ou d'une clé sur le dispositif d'entrée.

6. Pompe péristaltique d'injection intraveineuse selon la revendication 1, dans laquelle à chaque nom d'application mémorisé dans la mémoire sont affectés un code correspondant et un débit maximum spécifique d'injection du fluide et un volume maximum d'injection associés à ce nom d'application.

7. Pompe péristaltique d'injection intraveineuse selon la revendication 6, dans laquelle un opérateur peut sélectionner un nom d'application en sélectionnant son code correspondant par l'intermédiaire du dispositif d'entrée.

8. Pompe péristaltique d'injection intraveineuse selon la revendication 2, comprenant en outre :
des moyens pour produire un message d'erreur lorsque les moyens de décision décident que les valeurs modifiées des paramètres se situent à l'extérieur de la gamme déterminée pour le nom d'application sélectionné.
